# EUROPÄISCHE PATENTSCHRIFT

(11) **EP 2 630 938 B1**
(45) Veröffentlichungstag und Bekanntmachung des Hinweises auf die Patenterteilung: **17.06.2015**
(21) Anmeldenummer: 13000803.0
(22) Anmeldetag: 16.02.2013
(51) Int. Cl.: A61F 5/56

(54) **Vorrichtung zur Verbesserung der Atmung einer Person**
Device for improving the respiration of a person
Dispositif destiné à améliorer la respiration d'une personne

(30) Priorität: 23.02.2012 DE 102012003564
(43) Veröffentlichungstag der Anmeldung: 28.08.2013
(73) Patentinhaber: Scheffel, Bernd, 80999 München (DE)
(72) Erfinder: Scheffel, Bernd, 80999 München (DE)

(56) Entgegenhaltungen:
- WO-A2-2005/013867
- DE-A1-102008 007 281
- US-B1- 6 418 933

## Beschreibung

### Technisches Gebiet der Erfindung

Diese Erfindung betrifft allgemein orale Hilfsmittel und insbesondere eine Vorrichtung zur Verbesserung der Atmung eines Benutzers.

### Hintergrund

Es ist bekannt, dass die Vorverlagerung des Unterkiefers zur Abhilfe bei obstruktiven Atmungsstörungen erfolgreich eingesetzt wird. Anwendungen sind bekannt bei Schlafapnoe und Schnarchen.
Therapieerfolge wurden vor allem mit solchen Vorrichtungen erreicht, welche für den Benutzer speziell angefertigte Schienen für den oberen und unteren Zahnbogen aufweisen.

Solche Systeme benötigen zwischen den beiden Schienen Koppelelemente, welche für die zur Therapie erforderliche Unterkieferprotrusion bewirken. Bei diesen Koppelelementen gibt vielerlei Ausführungsformen.

Vorrichtungen, die innerhalb des Zahnbogens getragen werden, beineinträchtigen stark den Komfort des Benutzers, stören das Sprechen und Schlucken, können zur erhöhter Speichelbildung führen. Beispiele für solche Ausführungen mit Koppelelementen innerhalb des Zahnbogens sind in DE20319088U1, EP1312319A2 beschrieben.

Weitere Vorrichtungen mit Anordnung innerhalb des Zahnbogens, labial, wie z.B. beschrieben in DE69501111T2, WO2005013867A2, US6305376B1 erlauben nur geringe Mundöffnung. Soweit Verstelleinrichtungen für die Protrusion vorgeschlagen werden, bedeuten diese zusätzliche Beeinträchtigung des Tragekomforts, da diese einen Teil des Zungenfreiraumes beanspruchen. Da der Weg der Zunge zu den Lippen versperrt ist, kann der Benutzer auch nicht die Lippen mit der Zunge befeuchten. Eine wichtige Eigenschaft für das Wohlbefinden fehlt daher.

Ist die Anordnung der Koppelelemente außerhalb des Zahnbogens, sowie labial, wie in DE112009001742T5 gezeigt, so ist ebenso nur geringe Mundöffnung möglich. Außerdem kann der Mund ganz nicht verschlossen werden, was zu unerwünschter Mundatmung führt. So trifft hier zu, dass Sprechen behindert wird und dass der Weg der Zunge zu den Lippen versperrt ist und der Benutzer nicht die Lippen mit der Zunge befeuchten kann.

Labiale, außerhalb des Zahnbogens angeordnete Koppelemente, wie in DE102008007281A1 gezeigt, bauen weniger voluminös, wenn auf eine komplexe Verstelleinrichtung für die Protrusion verzichtet wird. Als Koppelelement zwischen beiden Zahnschienen dient ein gebogenes Führungselement, dessen Enden fest mit beiden Seiten der zweiten Zahnschiene verbunden sind. Das Öffnen des Mundes ist beschränkt. Beim Versuch den Mund weiter zu öffnen, entstehen abziehende Kräfte, welche die Schienen von den Zahnbögen entfernen. Damit geht die Wirkung des Systems verloren.

Eine häufig vorgeschlagene, weitere Art der Anordnung von Koppelelementen weist 2 Koppelelemente und 4 Gelenke außerhalb des Zahnbogens bukkal auf.

Bei den Vorrichtungen nach CA2694017, DE202007007760U1, DE000029506512U1, DE202008011841U1 wirkt zur Protrusion eine Zugkraft auf die Koppelelemente, da diese im anterioren Bereich der Oberkieferschiene und im posterioren Bereich der Unterkieferschiene angreifen. Die Koppelelemente sind teilweise als starre Stäbe ausgeführt, teilweise auch elastisch, teleskopartig, teilweise über Spindel-Mechanik längenverstellbar. Solche Lösungen sind voluminös, also wenig komfortabel zu tragen, mechanisch relativ aufwendig, teuer in der Herstellung und schlecht zu reinigen. Bei den erwähnten Vorrichtungen mit Zugkraftübertragung führt die Kinematik beim Öffnen des Mundes zur Erhöhung der Protrusion. Das kann bis zur Sperre gehen, d.h. der Mund kann ab einer gewissen Öffnung nicht weiter geöffnet werden. Beim Versuch den Mund weiter zu öffnen, entstehen abziehende Kräfte, welche die Schienen von den Zahnbögen entfernen. Damit geht die Wirkung des Systems verloren. Versuche diese Nachteile zu beheben sind in den Vorschlägen für längenveränderliche Koppelelemente gemacht worden. Dennoch ist es für den Benutzer unangenehm, wenn das Öffnen des Mundes behindert oder erschwert wird.

Bei den Vorrichtungen nach EP1972311A1, EP2081525B1, US6012920, US6526982B1 übertragen die Koppelelemente eine Druckkraft. Hier greifen die Koppelelemente im posterioren Bereich der Oberkieferschiene und im anterioren Bereich der Unterkieferschiene an.
Bei dieser Anordnung ist das das Öffnen des Mundes leichter, weniger begrenzt. Die Kinematik bewirkt beim Öffnen eine Verringerung der Protrusion des Unterkiefers. Die bukkale Anordnung der Koppelelemente hat eine kurze Baulänge zur Folge. Ein Koppelelement beschreibt einen Kreisbogen um den Gelenkpunkt an der Oberkieferschiene. Infolge des Kreisbogens mit kleinem Radius entsteht schon bei relativ geringem Öffnungswinkel ein starker Verlust an Protrusion. Um diesen Nachteil zu beheben wird in EP1972311A1 eine Längenanpassung mit Teleskop und Feder vorgeschlagen. Da jedoch die Federkraft zu einer unerwünschten Mundöffnung führt, ist zusätzlich eine Verschlusseinrichtung erforderlich. Sie hält den Mund geschlossen, worauf aber die Freiheit der Unterkieferbewegung verloren geht. Der Benutzer bekommt ein Gefühl "wie zugeschraubt".

Weiter sind Vorrichtungen gemäß DE102007050309B3 bekannt, mit 2 Koppelelementen, welche nur an den dorsalen Enden der Ober- und Unterkieferschienen befestigt sind. Sie weisen keine Gelenke auf, sondern sind fest mit den Schienen verbunden. Sie reagieren durch ihre eigene elastische Verformung auf das Öffnen des Mundes. Das führt auch zu einer Einschränkung der Mundöffnung und bewirkt abziehende Kräfte an beiden Schienen.

Eine ähnliche Lösung wird in DE102008051221A1 vorgeschlagen. Sie entspricht im Aufbau der vorgenannten Lösung, weist aber zusätzlich je 1

Gelenk in dem Koppelelement auf. Im Prinzip gilt für die Kinematik das Gleiche wie für Ausführungen ohne Gelenk. Bei Kieferbewegung entstehen auch hier Kräfte, welche versuchen, die Schienen von den Zahnbogen abzuziehen.

Die Anforderungen für die erfolgreiche Verbreitung von Protrusionsschienen werden von allen bekannten Vorrichtungen nur teilweise erfüllt.
Bisherige Mängel sind:
Mangelnder Komfort beim Tragen solcher Schienen,
mangelnde freie Beweglichkeit des Unterkiefers lateral und in der Sagitalebene, mangelnde Freiheit des Zungenraumes von technischen Bauteilen, Hindernisse bei Schließen der Lippen,
keine, oder umständliche Optimierbarkeit der Protrusion durch den Benutzer, eingeschränkte Funktionsdauer wegen empfindlicher Feinmechanik, erschwerte Pflege wegen komplizierter Formen,
hohe Herstellkosten wegen aufwendiger, komplizierter Einzelteile.

Die Aufgabe zur Behebung solcher Mängel wird durch die im Patentanspruch 1 aufgeführten Merkmale gelöst.
Wesentliche, mit der Erfindung erzielte Vorteile bestehen darin, dass diese ein einfaches, kostengünstig herstellbares, ohne Werkzeuge auswechselbares Koppelelement für obere und untere Schiene aufweist. Die Kinematik aus Schienen und Koppelelement erlaubt eine großzügige Beweglichkeit des Unterkiefers, besonders wichtig für Compliance Bruxismus, bei Niesen oder Gähnen.
Vorteilhafte Ausgestaltung der Erfindung sind in den Patentansprüchen 2 bis 12 angegeben. Verfahren zum Auswechseln des Koppelelementes sind in den
Patentansprüchen 13 und 14 angegeben. Ein Verfahren zum Überprüfen der Wahl der richtigen maximalen Größe des Koppelelementes ist in Anspruch 15 aufgezeigt.

### Kurze Beschreibung der Zeichnungen

Ein Ausführungsbeispiel der Erfindung ist in den Zeichnungen dargestellt und wird im Folgenden näher beschrieben.
Es zeigen:
Fig.1 ein Beispiel einer Vorrichtung zur Verbesserung der Atmung als isometrische Ansicht bei leicht geöffnetem Mund ;
Fig.2 ein Beispiel einer Vorrichtung zur Verbesserung der Atmung als Seitenansicht bei geöffnetem Mund ;
Fig.3 ein Beispiel einer Vorrichtung zur Verbesserung der Atmung als eine Seitenansicht mit Überlagerung der Darstellungen bei geöffnetem Mund und geschlossenem Mund;
Fig.4.1 bis 4.4 ein Beispiel einer Vorrichtung zur Verbesserung der Atmung als Ansichten einer Unterkieferschiene, wobei dargestellt sind:

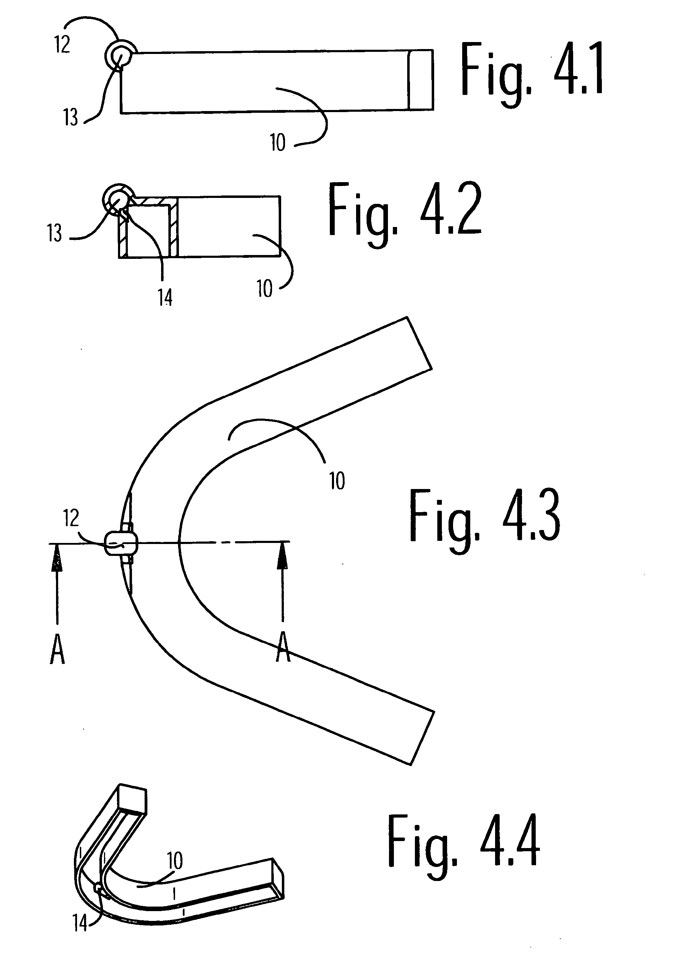

   Fig.4.1 eine Seitenansicht,
   Fig.4.2 einen Schnitt,
   Fig.4.3 eine Draufsicht,
   Fig.4.4 eine isometrische Ansicht;

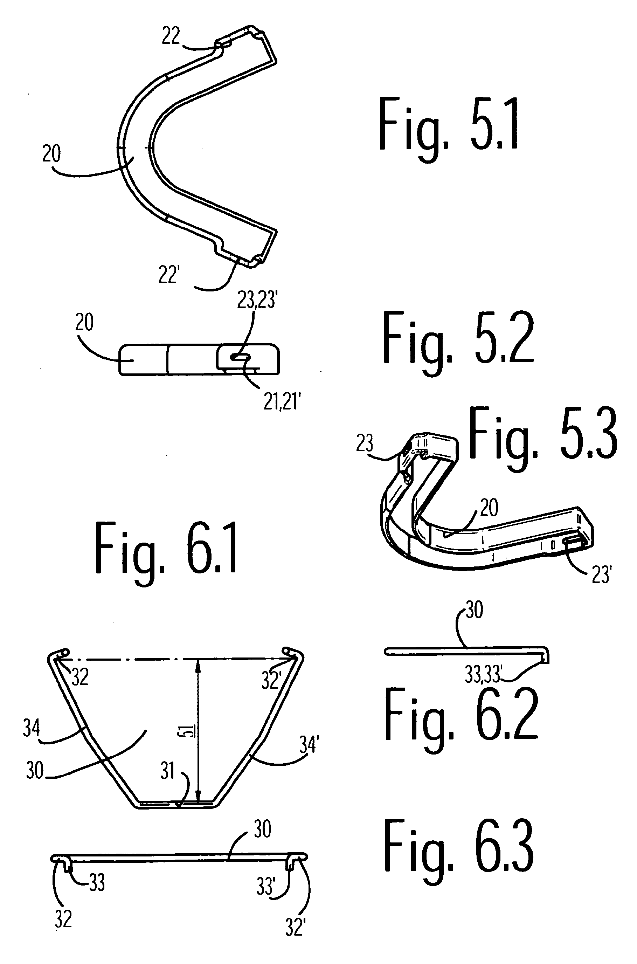

Fig.5.1 bis Fig 5.3 ein Beispiel der Vorrichtung zur Verbesserung der Atmung mit Ansichten einer Oberkieferschiene, wobei dargestellt sind:
   Fig.5.1 eine Draufsicht,
   Fig.5.2 eine Seitenansicht,
   Fig.5.3 eine isometrische Ansicht;
Fig.6.1bis Fig.6.3 ein Beispiel der Vorrichtung zur Verbesserung der Atmung mit Ansichten eines Koppelelementes wobei dargestellt sind:
   Fig.6.1 eine Ansicht,
   Fig.6.1 eine Seitenansicht,
   Fig.6.1 eine isometrische Ansicht;

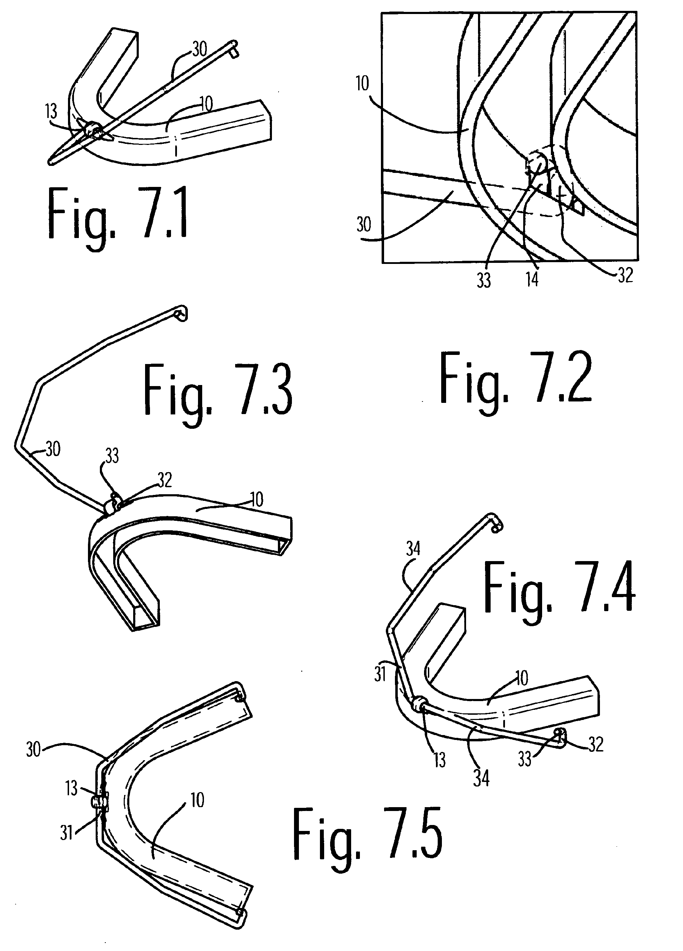

Fig. 7.1bis Fig.7.5 Beispieldarstellungen vom Verfahren zur Montage der Vorrichtung mit Zusammenfügen von Koppelelement und Unterkieferschiene, wobei dargestellt sind:
   Fig.7.1 eine isometrische Ansicht mit dem Einführen der ersten Abwinkelung,
   Fig.7.2 eine isometrische Ansicht von der inzisalen Seite,
   Fig.7.3 eine isometrische Ansicht nach dem Einführen der zweiten Abwinkelung,
   Fig.7.4 eine isometrische Ansicht nach dem Durchschieben eines Schenkels des Koppelelementes,
   Fig.7.5 eine Draufsicht im Zustand der kompletten Montage von Koppelelement und Unterkieferschiene;

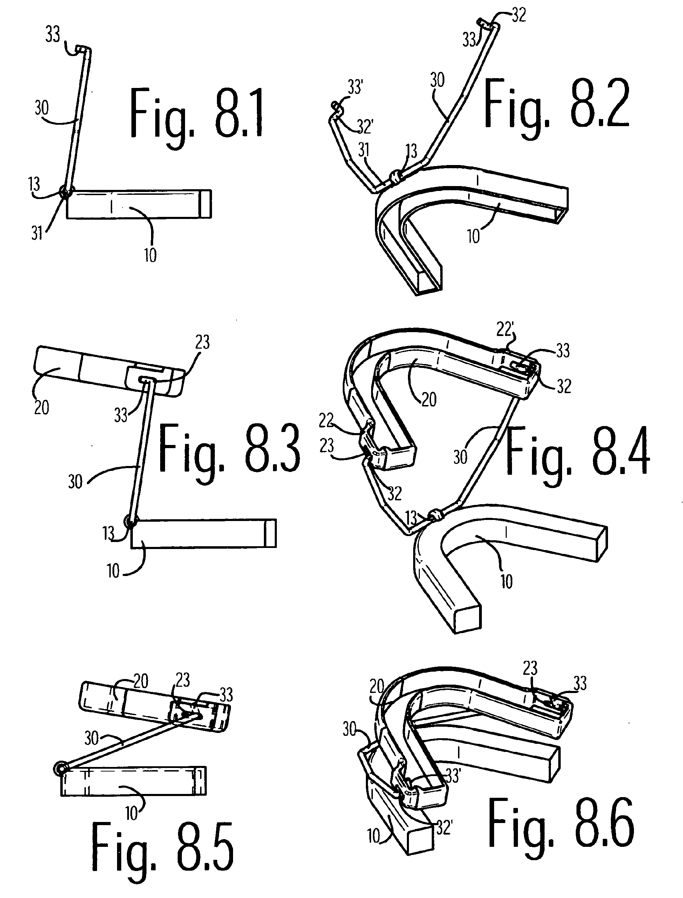

Fig.8.1bis Fig.8.6 Beispieldarstellungen zum Verfahren zur Montage der Vorrichtung mit Zusammenfügen von Koppelelement und Oberkieferschiene, wobei dargestellt sind:
   Fig.8.1 eine Seitenansicht von Unterkieferschiene und Koppelelement zu Beginn der Montage der Oberkieferschiene;
   Fig.8.2 eine isometrische Ansicht von Unterkieferschiene und Koppelelement zu Beginn der Montage der Oberkieferschiene;
   Fig.8.3 eine Seitenansicht von Oberkieferschiene und Koppelelement in der Position für das Verbinden;
   Fig.8.4 eine isometrische Ansicht von Oberkieferschiene und Koppelelement in der Position für das Verbinden;
   Fig.8.5 eine Seitenansicht von Unterkieferschiene und Koppelelement nach fertiger Montage im Zustand wie bei geöffnetem Mund;
   Fig.8.6 eine isometrische Ansicht von Unterkieferschiene und Koppelelement nach fertiger Montage im Zustand wie bei geöffnetem Mund.

### Beschreibung von Beispielausfürungsformen

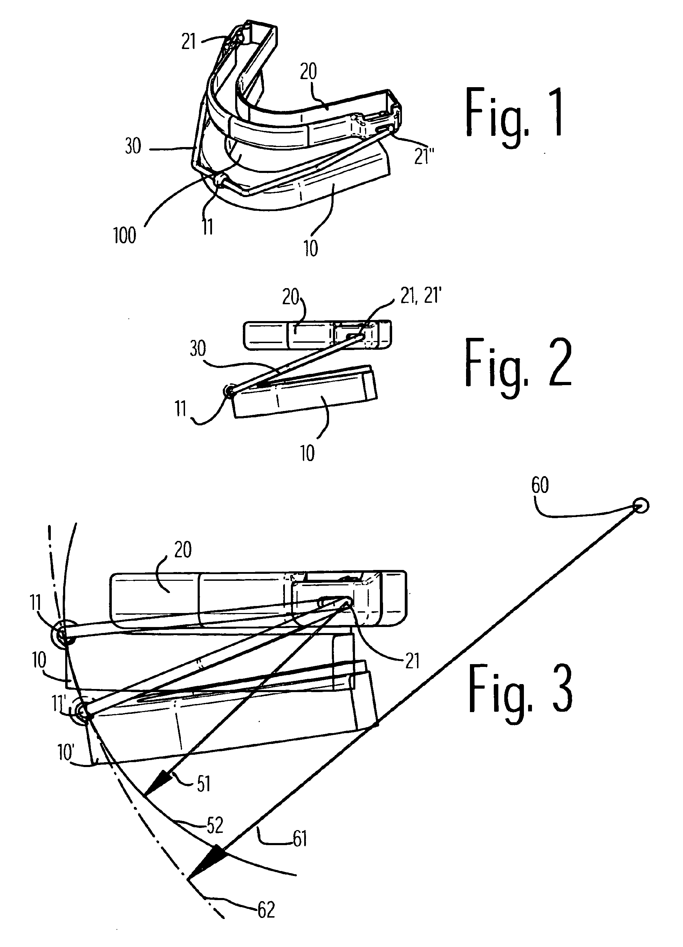

Fig.1 zeigt eine Beispielvorrichtung 100 zur Verbesserung der Atmung des Benutzers bei geschlossenem Mund. Die Vorrichtung 100 kann benutzt werden um Atemstörung beim Schlaf, wie Schnarchen oder Schlafapnoe zu behandeln, indem der Unterkiefer relativ zum Oberkiefer in einer vorgelagerten (protrutierten) Position gehalten wird. Dadurch werden die Atemwege im Bereich des Zungengrundes freigehalten. Während der Behandlung, im Schlaf, verbleibt die Vorrichtung 100 innerhalb des Mundes des Benutzers. Die Vorrichtung 100 umfasst die untere Zahnschiene 10 die geeignet ist, zumindest einige Zähne der unteren Zahnreihe des Benutzers zu umhüllen und eine obere Zahnschiene 20, die geeignet ist, zumindest einige Zähne der oberen Zahnreihe des Benutzers zu umhüllen. Die Zahnschienen 10 und 20 sind vorzugsweise mit Abformungen der Zähne versehen. Beispielhaftes Material für die Zahnschienen 100 ist gesundheitlich unbedenklicher Copolyester, sowie thermoplastisches Polyurethan, welche komfortables Tragen ermöglichen. In der gezeigten Ausführung sind die Zahnschienen 10 und 20 über ein Koppelement 30 verbunden. Dazu weist die untere Zahnschiene 10 einen vorderen Verbindungspunkt 11 labial am Scheitel im Bereich der vorderen Zähne auf. Der Verbindungspunkt 11 bildet für das Koppelelement 30 ein Lager für Drehung und laterale Verschiebung.

Für den Anschluss des Koppelelementes 30 an der oberen Zahnschiene 10 weist die Zahnschiene 20 zwei hintere Verbindungspunkte 21 und 21' bukkal im Bereich der posterioren Zähne auf.

Fig.2 zeigt eine Seitenansicht eines Beispieles bei leicht geöffnetem Mund. Das Koppelelement 30, ausgehend von den hinteren Verbindungspunkten 21 und 21' an der oberen Zahnschiene 20, verläuft zu beiden Seiten der Zahnbögen zum vorderen Verbindungspunkt 11 an der unteren Zahnschiene 10.

Die Kinematik beim Öffnen des Mundes wird anhand Fig.3 weiter erläutert. Beim Öffnen des Mundes folgt der Verbindungspunkt 11 an der unteren Zahnschiene 10 einem Bogen 52 mit dem Radius 51 um dem Mittelpunkt 21 (21') dem Verbindungspunkt an der Schiene des Oberkiefers. Dieser Radius 51 ist nur von den Maßen des Koppelelementes 30 abhängig. Ein gedachter Bogen 62 für konstante Protrusion verläuft um den Mittelpunkt 60 im Kiefergelenk und besitzt den Radius 61. Je mehr sich die Bögen 52 und 62 gleichen, umso mehr bleibt beim Öffnen die Protrusion konstant. Dann geht beim Öffnen des Mundes wenig von der Vorverlagerung des Unterkiefers verloren. Im Vergleich mit bekannten Vorrichtungen, ist der Radius 51 der erfindungsgemäßen Vorrichtung maximal. Möglich ist das wegen der maximalen vorderen Lage des Verbindungspunktes 11 an der unteren Zahnschiene 10 und der maximal dorsalen Lage der Verbindungspunkte 21(21') an der oberen Zahnschiene 20. Daher weist die erfindungsgemäße Vorrichtung nur geringen Verlust an Protrusion auf wenn der Mund geöffnet wird. Außerdem ist das Öffnen des Mundes ein keiner Weise durch die Vorrichtung eingeschränkt.

Die Schiene für den Unterkiefer besitzt im gezeigten Beispiel Fig. 4.1 den Verbindungspunkt 11 in einer Ausformung 12 mit einer lateralen Öffnung 13. Die Öffnung 13 kommuniziert mit der Öffnung 14, welche als Durchbruch zum Innenbereich der Schiene 10 in Richtung der Schneidkanten der Zähne (inzisal) ausgebildet ist. Diese inzisale Öffnung 14 ermöglicht das Montieren der Abwinkelungen (32, 33) des Koppelelementes. Im Schnittbild gemäß Fig. 4.2, welches durch die in Fig. 4.3 gezeigte Ansicht erzeugt wurde, ist die inzisale Öffnung 14 und ihr Zusammengehen mit der lateralen Öffnung 13 zu sehen. Einen weiteren Blick auf die inzisale Öffnung zeigt die Fig. 4.4. Die untere Schiene 10 ist mit bekannten Verfahren einfach herstellbar und leicht zu pflegen.

Ein Beispiel für die Ausbildung der Schiene für den Oberkiefer zeigen Fig. 5.1 bis 5.3. In der Draufsicht Fig. 5.1 erkennt man die Ausformungen 22 und 22'. In der Seitenansicht Fig. 5.2 sind die Ausformungen 22,22' mit den schlitzförmigen Öffnungen 13 und 13' dargestellt. In diesem Beispiel erstrecken sich die Schlitze parallel zu Okklusionsebene. Die Verbindungspunkte 21 und 21' befinden sich im Kreismittelpunkt am dorsalen Ende der schlitzförmigen Öffnungen 23 und 23'.

Die obere Schiene 20 ist ein sehr einfaches Bauteil, sowohl in der Herstellung, als auch für die Pflege, wie aus Fig. 5.3 leicht zu ersehen ist.

Das im Wesentlichen u-förmige Koppelelement 30 ist in Fig. 6.1 wiedergegeben. In diesem Beispiel handelt es sich dabei um ein einfaches Drahtbiegeteil. Zum Zwecke der einfachen Herstellung ist es als symmetrisches Polygon ausgeführt, welches nach den Maßen der zugehörigen Zahnschienen hergestellt wird. Vom Scheitel 31 des Koppelelementes 30 gehen die Schenkel 34 (34') aus und laufen bis zu einer Abwinkelung 32 (32'). Diese Abwinkelung 32 (32') dient als Lagerungsachse im Schlitz 23 (23') in der oberen Zahnschiene 20. Auf diese Abwinkelung 32 (32') steht senkrecht eine weitere Abwinkelung 33 (33'), Fig. 6.2 und Fig. 6.3. Ihr Aufgabe ist es das Koppelelement 30 gegen Herauslösen des aus der oberen Schiene 20 zu verriegeln. Zum Zwecke der benutzergerechten Einstellung der Protrusion können verschieden große Koppelelemente angefertigt werden. Sie unterscheiden sich im Radius 51 entsprechend den Abständen zwischen dem Steg 31 und den Abwinkelungen 32 (32'). Durch Auswahl und Montage der geeigneten Größe kann dann die gewünschte Protrusion des Unterkiefers eingestellt werden.

Mit diesen einfachen Bauteilen, der unteren Schiene 10, der oberen Schiene 20 und dem Koppelelement 30 wird durch einfache Montage eine funktionsfähige Vorrichtung zusammengestellt. Zu Beginn der Montage wird das Koppelelement 30 in die untere Schiene 10 eingesetzt. Wie aus Fig. 7.1 hervorgeht, wird das Ende des Koppelementes in die laterale Öffnung 13 eingeführt, verdreht und vorgeschoben, wobei es mit seinen Abwinkelungen teilweise durch die inzisale Öffnung 14 tritt und schließlich nach weiterem Drehen und Vorschieben aus dem gegenüberliegenden lateralen Ende der Öffnung 23 hervor tritt (Fig. 7.2 und Fig. 7.3). Hierauf kann das Koppelelement 30 mit seinem Schenkel 34 weiter durch die Öffnung 13 verschoben werden. Dieser Vorgang ist in Fig. 7.4 dargestellt. Wenn der Scheitel 31 sich in der Öffnung 13 befindet (Fig. 7.5) ist die Montage von unterer Schiene und Koppelelement fertig.

In den Zeichnungen Fig. 8.1 bis 8.6 wird die bisher montierte Baugruppe aus unterer Schiene 10 und Koppelelement 30 durch Montage der oberen Schiene 20 vervollkommnet. Dazu wird die untere Schiene mit Koppelelement in eine Lage entsprechend Fig. 8.1 und 8.2 gebracht. Daraufhin kann die obere Schiene so zwischen den Abwinkelungen 33 des Koppelelementes 30 positioniert werden, dass die Abwinkelungen 33 und die Schlitze 23 der oberen Schiene deckungsgleich sind (Fig. 8.3). Dann werden die Abwinkelungen 33 (33') durch die Schlitze 23 gedrückt, bis sie auf der Innenseite der Ausformungen 22 (22') heraustreten. (Fig. 8.4). Anschließend wird die obere Schiene 20 auf die untere Schiene 10 abgesenkt. Dabei verdreht sich das Koppelelement 30 gegenüber der oberen Schiene 20. Da nun die Abwinkelungen 33 und die Schlitze 23 nicht mehr deckungsgleich sind, kann sich das Koppelelement 30 nicht mehr von der oberen Schiene 20 lösen. Das trifft auch zu, wenn der Benutzer die Vorrichtung in den Mund eingesetzt hat und den Mund öffnet, denn die Öffnung des Mundes reicht nicht aus, um Schlitze 23 und Abwinkelung 33 zur Deckung zu bringen.

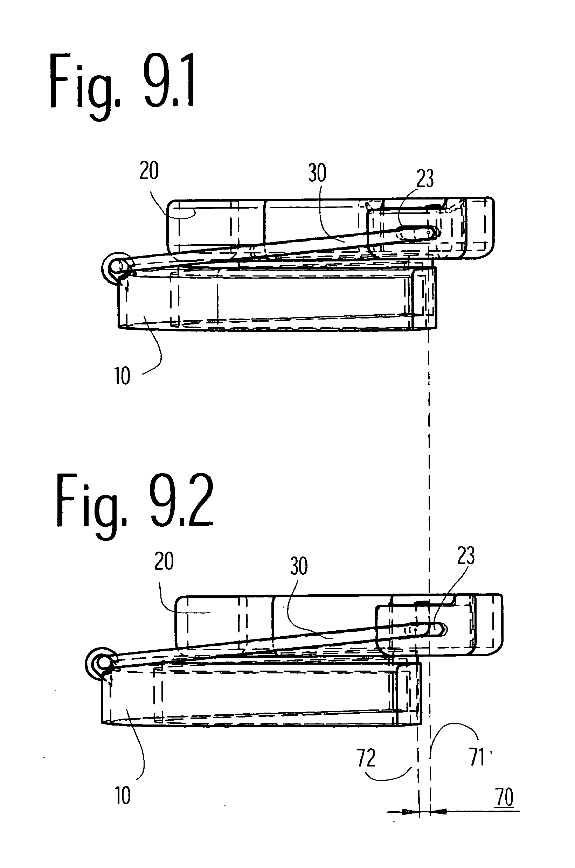

Die Schlitze 23 in der oberen Schiene haben noch eine weitere, sehr vorteilhafte Funktion. Sie erlauben eine Überprüfung der eingestellten Protrusion des Unterkiefers. Aus medizinischen Gründen soll eine solche Vorrichtung die maximal mögliche Protrusion nicht voll ausnutzen.
Bei protrutiertem Unterkiefer, wie in Fig. 9.1 gezeigt, liegt das Koppelelement 30 mit seiner Abwinkelung 32 (32') am dorsalen Ende des Schlitzes 23 an. Bei richtiger Wahl der Größe des Koppelgliedes soll noch eine Reserve an Protrusion bestehen. Um diese zu testen (Fig. 9.2), wird versucht den Unterkiefer durch Muskelanspannung noch weiter nach vorn zu schieben. Bewegt sich die Abwinkelung 32 (32') des Koppelelementes 30 von seiner Lage 71 am dorsalen Ende des Schlitzes 23 zum vorderen Ende des Schlitzes 72, so ist eine Reserve 70 an Protrusion vorhanden. Gelingt dies nicht, so ist das Koppelelement zu groß und muss durch die nächst kleinere Größe ersetzt werden.

## Patentansprüche

1. Vorrichtung zur Verbesserung der Atmung eines Benutzers durch Protrusion des Unterkiefers, mit:
- einer ersten Zahnschiene (10), die geeignet ist, zumindest einige Zähne der unteren Zahnreihe des Benutzers zu umhüllen;
- und einer zweiten Zahnschiene (20), die geeignet ist, zumindest einige Zähne der oberen Zahnreihe des Benutzers zu umhüllen;
- und mindestens einem Koppelelement (30), welches die erste (10) Zahnschiene und die zweite Zahnschiene (20) verbindet
**dadurch gekennzeichnet,**
**dass** die Zahnschiene (10) für den Unterkiefer einen vorderen Verbindungspunkt (11) labial aufweist und
die Zahnschiene für den Oberkiefer (20) zwei hintere Verbindungspunkte (21,21') bukkal, im Wesentlichen an ihren beiden posterioren Enden aufweist;
**dass** ein Koppelelement (30) eine im Wesentlichen U-förmige Gestalt aufweist, wobei dessen Scheitel mit dem vorderen Verbindungspunkt (11) an der Zahnschiene (10) für den Unterkiefer ein Gelenk bilden und
**dass** die dorsalen Enden (32) des Koppelelements (30) mit den hinteren Verbindungspunkten der Zahnschiene (20) für den Oberkiefer ein Gelenk bilden.

2. Vorrichtung nach Anspruch 1
**dadurch gekennzeichnet**,
und dass die Protrusion von der Größe (35) des Koppelelementes abhängig ist und dass das Auswechseln des Koppelelementes (30) ohne Zuhilfenahme von Werkzeugen möglich ist und dadurch eine einfache Veränderung der Protrusion erreicht wird.

3. Vorrichtung nach Anspruch 1
**dadurch gekennzeichnet,**
**dass** der vordere Verbindungspunkt (11) für den Unterkiefer mit einer Ausformung (12) versehen ist, die eine durchgehende, lateral gerichteten Öffnung (13) aufweist, durch die der Scheitel (31) des Koppelelementes (30) verläuft und mit ihm ein Dreh-Schiebegelenk bildet.

4. Vorrichtung nach Anspruch 2
**dadurch gekennzeichnet,**
**dass** die Zahnschiene(10) für den Unterkiefer, im Bereich der lateral gerichteten Öffnung(13) auch eine inzisale Ausnehmung(14) aufweist, welche mit der lateralen Öffnung (13) kommuniziert.

5. Vorrichtung nach Anspruch 1
**dadurch gekennzeichnet,**
**dass** das im Wesentlichen U-förmige Koppelelement (30) an seinen dorsalen Enden je eine mesial gerichtete erste Abwinkelung(32, 32') trägt.

6. Vorrichtung nach Anspruch 4
**dadurch gekennzeichnet,**
**dass** auf jede der mesialen ersten Abwinkelungen(32, 32') des U-förmigen Koppelelementes (30) je eine zweite Abwinkelung(33, 33') folgt, welche im Wesentlichen senkrecht auf die erste Abwinkelung(32, 32') steht.

7. Vorrichtung nach Anspruch 6
**dadurch gekennzeichnet,**
**dass** die bukkalen Verbindungspunkte(21,21') an der Schiene(20) für den Oberkiefer laterale, schlitzartige Öffnungen(23, 23') aufweisen, mit einer Länge des Schlitzes mindestens so groß, wie die Länge der zweiten Abwinkelung(33, 33') des Koppelelementes(30).

8. Vorrichtung nach Anspruch 7
**dadurch gekennzeichnet,**
**dass** die schlitzartigen Öffnungen(23, 23') im Wesentlichen parallel zur Oklusionsebene verlaufen

9. Vorrichtung nach Anspruch 1
**dadurch gekennzeichnet,**
**dass** das im Wesentlichen U-förmige Koppelelement (30) einstückig aus Draht gebildet ist.

10. Vorrichtung nach Anspruch 1
**dadurch gekennzeichnet,**
**dass** die im Wesentlichen U-förmige Gestalt des Koppelelementes (30) durch eine mehreckige Kurve gebildet wird.

11. Vorrichtung nach Anspruch 1
**dadurch gekennzeichnet,**
**dass** der vordere Verbindungspunkt (11) für den Unterkiefer durch eine labiale Ausformung(12) der Unterkiefer-Zahnschiene(10) gebildet ist

12. Vorrichtung nach Anspruch 6
**dadurch gekennzeichnet,**
**dass** die Verbindungspunkt (21) an der Oberkiefer-Zahnschiene durch bukkale Ausformungen(22, 22') der Zahnschiene(20) gebildet sind

13. Verfahren zur Montage der Vorrichtung nach Anspruch 3 und 5 und 7,
**dadurch gekennzeichnet,**
**dass** zum Zusammenfügen des Koppelelementes(30) mit der Unterkieferschiene(10) die zweite Abwinkelung (33) des Koppelelementes(30) in die der laterale Öffnungen(13) im Verbindungspunkt(11) der Unterkieferschiene(10) eingeführt wird und dass dann das Koppelelement(30) unter Vorschub so verdreht wird, bis die Abwinkelung (33) durch die in inzisale Ausnehmung(14) tritt und dann wiederum unter Vorschub so verdreht wird, dass die Abwinkelung(33) durch die gegenseitige laterale Öffnung(13) des Verbindungspunktes(11) der Unterkieferschiene(10) austritt, worauf das u-förmige Koppelement(30) weiter verschoben wird, bis es die gewünschte Funktionslage in der Unterkieferschiene(10) einnimmt.

14. Verfahren zur Montage der Vorrichtung nach Anspruch 5 und 6 und 7,
**dadurch gekennzeichnet,**
**dass** zum Zusammenfügen des Koppelelementes(30) mit der Oberkieferschiene(20) diese so ausgerichtet wird, dass sie zwischen den Enden(33, 33') des Koppelelementes(30) zu liegen kommt und die Schlitze(23, 23') deckungsgleich mit den zweiten Abwinkelungen (33,33') des Koppelelementes(30) sind und dass dann die Abwinkelungen (33, 33') durch die Schlitze(23, 23') in das Innere der Schiene(20) hindurchgeführt werden, bis ein Schwenken der Oberkieferschiene(20) gegenüber dem Koppelelement(30) möglich ist und dass nach diesem Schwenken die zweiten Abwinkelungen (33, 33') nicht mehr deckungsgleich mit den Schlitzen(23, 23') sind, sodass sich das Koppelelement nicht mehr von der Oberkieferschiene lösen kann.

15. Verfahren unter Verwendung der Vorrichtungen nach Anspruch 8,
**dadurch gekennzeichnet,**
**dass** eine Bestätigung für das Vorliegen der maximal zulässigen Größe des montierten Koppelelementes(30) dadurch erfolgt, dass bei relativ geschlossenem Mund eine Hin- und Herbewegung des Unterkiefers in der Größenordnung der Lange der schlitzartigen Öffnungen(23, 23') möglich ist.

## Claims

1. A device for improving the breathing of a user by means of protrusion of the lower jaw, comprising:
- a first dental rail (10) which is adapted to coat at least some of the teeth of the lower row of teeth of the user;
- and a second dental rail (20) which is adapted to coat at least some teeth of the upper row of teeth of the user;
- and at least one coupling element (30) which connects the first (10) and the second dental rail (20)
wherein in the first dental rail (10) for the lower jaw comprises a front labial connection point (11) and
the second dental rail (20) has two rear buccal connection points (21, 21'), positioned substantially at its two posterior ends,
wherein the coupling element (30) has a substantially U-shaped configuration, wherein an apex of the coupling element forms a joint with the front connecting point (11) on the first dental rail (10), and wherein
the posterior ends (32) of the coupling element (30) form a joint with the rear connection points of the second dental rail (20).

2. The device according to claim 1
wherein advancement of the lower yaw of the user is dependent on the size (35) of the coupling element(3o) and wherein the coupling element (30) is configured to be changed without the use of tools.

3. The device according to claim 1
wherein the front connection point (11) for the lower jaw has a protrusion (12) with a continuous, laterally directed opening (13) for reciving the apex (31) of the coupling element (30) and forming with it a turning sliding joint.

4. The device according to claim 2
wherein that the first dental rail (10) for the lower jaw, in the region of the laterally directed opening (13) is an incisal recess (14) that communicates with the lateral directed opening (13).

5. The device according to claim 1
wherein the substantially U-shaped coupling element (30) has posterior, mesial directed first bend (32, 32') on each end.

6. The device according to claim 4
wherein that on each of the mesial first bends (32, 32 ') of the U-shaped coupling element (30) a second bend (33, 33') on each bend that is substantially perpendicular to the first bend (32, 32 ').

7. The device according to claim 6
wherein the buccal connection points (21,21 ') have lateral slit-like openings (23, 23'), wherein a length of said openings is at least as great as the length of the second bend (33, 33') of the coupling element (30).

8. The device according to claim 7
wherein that the slot-like openings (23, 23') extend substantially parallel to an occlusion plane of the teeth.

9. The device according to claim 1
**characterized in**
**that** the substantially U-shaped coupling element (30) is integrally formed from wire.

10. The device according to claim 1
wherein the substantially U-shaped coupling element(30) is formed by a polygon.

11. The device according to claim 1
wherein the front labial connecting point (11) is formed a labial protrusion (12) of the first dental rail (10).

12. The device according to claim 1
wherein the buccal connection points (21, 21') on the second dental rail (20) are formed by buccal protrusions (22, 22 ') of the second dental rail

13. A method for assembling device of claim 3 and 5 and 7,
comprising the following steps:
for assembling the coupling element (30) with the first dental rail (10), inserting an angled end (33) of the coupling element (30) into the lateral directed opening (13) at the front, labial connection point (11) of first dental rail (10);
rotating the coupling element (30) under feed until the angled end (33) passes through the recess in an incisal opening (14) and further rotating the coupling element (30) under feed so that the angeled end(33) exits the lateral directed opening (13) at an opposite side of the connection point (11);
displacing the coupling element(30) further until the desired functional position in the dentel rail (10) is obtained.

14. A method of assembling the device of claim 5 and 6 and 7,
comprising the steps of:
aligning the second dental rail (20) between the ends (33, 33') of the coupling element (30) that at the assembling of the coupling element (30);
aligning the slit-like openings (23, 23') with the second angled ends (33,33') of the coupling element (30) ;
passing the angled ends (33, 33') through the slit-like openings (23, 23') into an interior of the second rail (20) until a pivoting of the second rail (20) relative to the coupling element (30) is possible, such that after that pivoting, the angled ends (33, 33') are no longer aligned with the slit-like openings (23, 23') so that the coupling element (30) is not disconnectable from of the second dental rail.

15. A method using devices according to claim 8,
wherein the maximally permissible size of the mounted coupling element (30) is confirmed if a reciprocation of the lower jaw is possible within the length of the slit-like openings (23, 23') while the mouth is closed.

## Revendications

1. Orthèse pour l'amelioration du flux aérien dans les vois respiratoires supérieures par l'avancée mandibulaire, comprenant
- une premiere gouttière (10) destinée à recouvir au moins quelques dents de l'arcade mandibulaire ;
- et une seconde gouttière (20) destinée à recevoire au moins quelques dents de l'arcade maxillaires ;
- et au moins un élément de couplage (30) reliant la premiere gouttière (10) a la seconde gouttière (20),
**caractérisée en ce que** la premiere gouttière (10) comportent un point d'attache (11) labial et
la seconde gouttière (20) comportent pour l'essentiel deux points d'attache (21, 21') buccals a ses deux extrémités dorsales ;
**caractérisée en ce que** un élément de couplage (30) possèsant une forme U, **caractérisée en ce que** le sommet du élément de couplage et le point d'attache (11) de la premiere gouttière (10) réalisent une articulation et
**caractérisée en ce que** les deux bouts (32) de l'élément de couplage (30) et les deux points d'attache dorsales du seconde gouttière (20) réalisent une articulation.

2. Orthèse selon la revendication 1, **caractérisée en ce que** la protrusion est dependant de la largeur (35) du élément de couplage (30) et est possible d'echanger le élément de couplage sans outils et c'est pourquoi il est possible de changer la protrusion facilement.

3. Orthèse selon la revendication 1, **caractérisée en ce que** le point d'attache (11) de la premiere gouttière (10) a une formation (12) qui contienne un orifice (13) passant a la direction lateral, par qui passe le sommet (31) du élément de couplage (30) et les deux formant une articulation pivotante et coulissante.

4. Orthèse selon la revendication 2, **caractérisée en ce que** la premiere gouttière (10) a la zone de l'orifice(13) lateral, contienne une voie (14) inzisale qui communique avec le orifice (13) lateral.

5. Orthèse selon la revendication 1, **caractérisée en ce que** de l'élément de couplage (30) du forme U, les bouts dorsales étant pliés a angle (32, 32') en direction mesial.

6. Orthèse selon la revendication 4, **caractérisée en ce que** de l'élément de couplage (30) du forme U, comprenant bouts dorsales (32, 32') angles secondaire (33, 33') qui en essentiel sont perpenticulaire aux angles (32, 32') premieres.

7. Orthèse selon la revendication 6, **caractérisée en ce que** les deux points d'attache (21, 21') buccals du seconde gouttière comportent interstices (23, 23') dont au moins la longueur est si grande que la longeur du angles (33, 33') secondaires de l'élément de couplage (30).

8. Orthèse selon la revendication 7, **caractérisée en ce que** les interstices (23, 23') pour l'essentiel sont parallelé à la plaine d'occlussion.

9. Orthèse selon la revendication 1, **caractérisée en ce que** l'élément de couplage (30) est formé integrale de fil métallique.

10. Orthèse selon la revendication 1, **caractérisée en ce que** l'élément de couplage (30) pour l'essentiel d'une forme U est formé come un polygone.

11. Orthèse selon la revendication 1, **caractérisée en ce que** le point d'attache (11) de la premiere gouttière (10) est formé par une éminence (12) labiale a la premiere gouttière (10).

12. Orthèse selon la revendication 6, **caractérisée en ce que** les deux points d'attache (21, 21') de la seconde gouttière (20) sont formé par éminences (22, 22') buccales a la seconde gouttière (20).

13. Procédure pour la montage de l'orthèse selon la revendication 3 et 5 et 7, , pour assemblage du élément de couplage (30) et la premiere gouttière (10), poser un bout (33) plié du élément de couplage (30) dans l'orifice (13) lateral de la premiere gouttière (10), tourner et pousser l'élément de couplage (30) jusque le bout (33) plié se passe par l'orifice (14) et tourner et avancer encore a une maniere que le bout (33) sorte de l'orifice (13) a l'autre côté du point d'attache (11), déplacer l' élément de couplage (30) jusque a atteindre la position fonctional dans la premiere gouttière (10).

14. Procédure pour la montage de l'orthèse selon la revendication 5 et 6 et 7, orienter la seconde gouttière (20) entre les bouts (33,33') du élément de couplage (30), orienter les interstices (23,23') aux bouts pliés (33,33') du élément de couplage (30), passer les bouts pliés (33,33') par les interstices (23,23') a l'interieur de la seconde gouttière (20) jusque il es possibe de tourner la seconde gouttière (20) relativement au élément de couplage (30) et l'élément de couplage (30) et les deux ne sont pas détachable.

15. Procédure utilisant l'orthèse selon la revendication 8, **caractérisée en ce que** la largeur maximale du élément de couplage (30) est confirmé quand la reciprocation mandibulaire est possible entre la longeur des interstices (23, 23') quand la bouche est fermée.
